# EUROPÄISCHE PATENTSCHRIFT

(11) **EP 3 146 944 B1**
(45) Veröffentlichungstag und Bekanntmachung des Hinweises auf die Patenterteilung: **13.03.2019**
(21) Anmeldenummer: 16189941.4
(22) Anmeldetag: 21.09.2016
(51) Int. Cl.: A61F 5/05, A61F 5/058, A61F 5/01

(54) **HANDGELENKSORTHESE**
WRIST ORTHOTIC
ORTHESE DE POIGNET

(30) Priorität: 23.09.2015 DE 102015012320
(43) Veröffentlichungstag der Anmeldung: 29.03.2017
(73) Patentinhaber: Albrecht GmbH, 83071 Stephanskirchen (DE)
(72) Erfinder: Wolf, Michael, 83059 Kolbermoor (DE)
(74) Vertreter: Flach Bauer Stahl Patentanwälte Partnerschaft mbB

(56) Entgegenhaltungen:
- WO-A1-94/04104
- DE-U1-202004 005 876
- US-A- 3 631 542

## Beschreibung

Die Erfindung betrifft eine Handgelenksorthese gemäß dem Oberbegriff des Anspruchs 1.

Bekannte Handgelenksorthesen weisen einen am Unterarm befestigbaren proximalen Schienenarm, einen distalen Schienenarm, ein Drehgelenk zur gelenkigen Verbindung des distalen Schienenarms mit dem proximalen Schienenarm und eine am distalen Schienenarm befestigte Handauflageeinrichtung auf, auf welcher die Hand aufgelegt werden kann. Mittels des Drehgelenks kann der distale Schienenarm und damit die Handauflageeinrichtung relativ zum proximalen Schienenarm abgewinkelt und ggf. in bestimmten Winkelstellungen arretiert werden. Dies ermöglicht es, die Hand relativ zum Unterarm bei angelegter Handgelenksorthese zu bewegen oder in bestimmten Winkelstellungen zu arretieren.

Weiterhin gibt es auch Handgelenksorthesen in der Form von dynamischen Redressionsorthesen. Derartige Handgelenksorthesen weisen eine Krafterzeugungseinrichtung, üblicherweise in der Form einer Feder, auf, mit welcher der distale Schienenarm relativ zum proximalen Schienenarm in Extensions- oder Flexionsrichtung gedrängt wird. Derartige dynamische Handgelenksorthesen dienen der Behandlung von sowohl orthopädisch als auch neurologisch bedingten Gelenkskontrakturen. Durch eine kontinuierliche, dosierte Dauerzugbehandlung können derartige Handgelenksorthesen Bewegungsdefizite beseitigen und die Rückkehr in einen physiologischen Bewegungsablauf unterstützen. Eine weitere Indikation für dynamische Handgelenksorthesen sind Gelenksfehlstellungen und die Prävention einer erneuten Kontraktur nach einer Arthrolyse.

Aus der US 3,631,542 A ist eine Handgelenksorthese gemäß dem Oberbegriff des Anspruchs 1 bekannt. Diese Handgelenksorthese ist als dynamische Orthese ausgebildet und weist einen hydraulischen Aktuator auf, mit dem ein Fingerstützelement um ein Schwenklager geschwenkt werden kann, das zwischen dem Fingerstützelement und einem Handballenstützelement angeordnet ist.

Die DE 20 2004 005 876 U1 beschreibt eine Handgelenksorthese mit einem schalenförmigen Fingerstützelement, das ebenfalls mittels eines Gelenks mit einem Handballenstützelement schwenkbar verbunden ist.

Eine gelenkige Verbindung zwischen einem Fingerstützelement und einem Handballenstützelement ist auch aus der WO 94/04104 A1 entnehmbar, wobei dort eine zusätzliche Arretiereinrichtung in der Form eines Spindeltriebs vorhanden ist, mit dem die Winkelstellung zwischen dem Fingerstützelement und dem Handballenstützelement verändert und arretiert werden kann.

Der Erfindung liegt die Aufgabe zugrunde, eine Handgelenksorthese der eingangs genannten Art zu schaffen, die eine besonders einfache Anpassung der Handauflageeinrichtung an unterschiedliche Handstellungen und Arretierung des Fingerstützelements am Handballenstützelement ermöglicht.

Diese Aufgabe wird erfindungsgemäß durch eine Handgelenksorthese mit den Merkmalen des Anspruchs 1 gelöst. Vorteilhafte Ausführungsformen der Erfindung sind in den weiteren Ansprüchen beschrieben.

Bei der erfindungsgemäßen Handauflageeinrichtung ist das zweite Lagerteil der Drehlagereinrichtung mittels der Kraft einer Krafterzeugungseinrichtung in eine Verriegelungsstellung vorgespannt, in der das zweite Lagerteil in Druckkontakt mit dem ersten Lagerteil und relativ zum ersten Lagerteil drehverriegelt ist, wobei das zweite Lagerteil entgegen der Vorspannkraft der Krafterzeugungseinrichtung in eine Freigabestellung bewegbar ist, in der das zweite Lagerteil relativ zum ersten Lagerteil drehbar ist.

Mit Hilfe der erfindungsgemäßen Handgelenksorthese kann das Fingerstützelement in unterschiedliche Winkelstellungen relativ zum Handballenstützelement gebracht und in der gewünschten Drehposition arretiert werden. Hierdurch lässt sich die Winkelstellung der Finger relativ zum Handballen auf einfache Weise variieren. Die Fingerstellung kann damit bestmöglich den individuellen medizinischen Erfordernissen angepasst werden. Insbesondere ermöglicht diese Ausführungsform auf besonders einfache Weise ein werkzeugloses Verstellen des Fingerstützelements relativ zum Handballenstützelement, indem das zweite Lagerteil vom ersten Lagerteil weggezogen, in die gewünschte Stellung gedreht und anschließend wieder losgelassen wird, wodurch das Fingerstützelement automatisch relativ zum Handballenelement in der gewünschten Winkelstellung arretiert wird.

Gemäß einer vorteilhaften Ausführungsform trägt das am distalen Schienenarm befestigte erste Lagerteil das Handballenstützelement. Vorteilhafterweise ist hierbei das zweite Lagerteil fluchtend zum ersten Lagerteil angeordnet.

Gemäß einer vorteilhaften Ausführungsform weist die distale Schiene ein sich in ihrer Längsrichtung erstreckendes Langloch oder eine Mehrzahl von Bohrungen zur Befestigung des ersten Lagerteils in unterschiedlichen Abständen zum Drehgelenk auf. Hierdurch lässt sich auf einfache Weise der Abstand der gesamten Handauflageeinrichtung relativ zu demjenigen Drehgelenk, mit dem die distale und proximale Schiene miteinander verbunden sind, variieren.

Vorteilhafterweise sind das erste und zweite Lagerteil zumindest überwiegend zylinderförmig ausgebildet. Scharfe Kanten könnten hierdurch vermieden werden. Es ist jedoch auch ohne weiteres möglich, anders geformte Lagerteile zu verwenden, beispielsweise quaderförmige Lagerteile.

Vorteilhafterweise weisen das erste und zweite Lagerteil den gleichen Außendurchmesser auf. Dies ermöglicht auf einfache Weise einen stufenlosen Übergang zwischen dem Handballenstützelement und dem Fingerstützelement, wenn diese auf dem ersten bzw. zweiten Lagerteil befestigt sind.

Vorteilhafterweise besteht die Krafterzeugungseinrichtung aus einer Feder, beispielsweise einer Druckfeder. Die Krafterzeugungseinrichtung kann jedoch auch in anderer Weise ausgebildet werden und beispielsweise aus einem elastischen Zugband bestehen, das einerseits am ersten Lagerteil und andererseits am zweiten Lagerteil festgelegt ist.

Vorteilhafterweise ist das zweite Lagerteil längsverschiebbar an einem Führungsstift geführt, der am ersten Lagerteil befestigt ist und sich in das zweite Lagerteil hineinerstreckt. Vorteilhafterweise ist dieser Führungsstift fluchtend zur Längsachse des ersten und zweiten Lagerteils angeordnet. Ein derartiger Führungsstift bildet somit gleichzeitig den Achsbolzen, um den das zweite Lagerteil relativ zum ersten Lagerteil drehbar ist, als auch dasjenige Führungselement, längs dem das zweite Lagerteil verschoben werden kann.

Gemäß einer vorteilhaften Ausführungsform besteht der Führungsstift aus einer zentralen Schraube, die in das erste Lagerteil eingeschraubt ist und einen Schraubenkopf aufweist, der einen Anschlag für ein erstes Ende der Feder bildet, während ein zweites Ende der Feder im oder am zweiten Lagerteil abgestützt ist. Hierdurch lässt sich eine sehr einfache Drehlagereinrichtung mit wenigen Teilen realisieren.

Vorteilhafterweise ist das zweite Lagerteil hülsenförmig ausgebildet, wobei der Schraubenkopf und die Feder im Inneren des zweiten Lagerteils angeordnet sind. Die Feder und der Schraubenkopf können damit unsichtbar und geschützt innerhalb des zweiten Lagerteils angeordnet werden.

Gemäß einer vorteilhaften Ausführungsform umfasst die Arretiereinrichtung zum Arretieren des Fingerstützelements relativ zum Handballenstützelement in einer bestimmten Drehwinkelstellung eine Rasteinrichtung, die mindestens einen Stift oder Zahn aufweist, der am ersten oder zweiten Lagerteil angeordnet ist und in unterschiedlich positionierte Rastvertiefungen eingeführt werden kann, die am gegenüberliegenden Lagerteil angeordnet sind. Hierdurch lässt sich das zweite Lagerteil und damit das Fingerstützelement auf sehr einfache Weise in unterschiedlichen Drehwinkelstellungen am ersten Lagerteil verrasten.

Vorteilhafterweise sind die Rastvertiefungen in Umfangsrichtung des ersten oder zweiten Lagerteils in einem regelmäßigen Winkelabstand angeordnet. Beispielsweise können die Rastvertiefungen durch einen Lochkreis mit Löchern gebildet werden, die in einem Abstand von 15° angeordnet sind.

Die Erfindung wird nachfolgend anhand von Zeichnungen beispielhaft näher erläutert. Es zeigen:
- Fig. 1 :: eine räumliche, teilweise etwas schematische Darstellung der erfindungsgemäßen Handgelenksorthese schräg von oben ohne Gurtbänder,
- Fig. 2 :: die Handgelenksorthese von Fig. 1 schräg von unten,
- Fig. 3 :: eine schematische Draufsicht der am Arm eines Patienten angelegten Handgelenksorthese,
- Fig. 4 :: eine schematische Seitenansicht der Darstellung von Fig. 3, wobei sich das Fingerstützelement in einer ersten Winkelstellung befindet und ohne Polsterung dargestellt ist,
- Fig. 5 :: eine schematische Darstellung gemäß Fig. 4, wobei das Fingerstützelement in einer zweiten Winkelstellung dargestellt ist,
- Fig. 6 :: eine Explosionsdarstellung der Drehlagereinrichtung und des distalen Schienenarms,
- Fig. 7 :: einen Längsschnitt durch das zweite Lagerteil,
- Fig. 8 :: eine stirnseitige Ansicht des ersten Lagerteils,
- Fig. 9 :: einen Längsschnitt durch das erste Lagerteil, und
- Fig. 10 :: eine Draufsicht auf das am ersten Lagerteil befestigte Handballenstützelement und das am zweiten Lagerteil befestigte Fingerstützelement.

Im Folgenden wird die Erfindung anhand einer Handgelenksorthese beschrieben, die als dynamische Redressionsorthese ausgebildet ist.

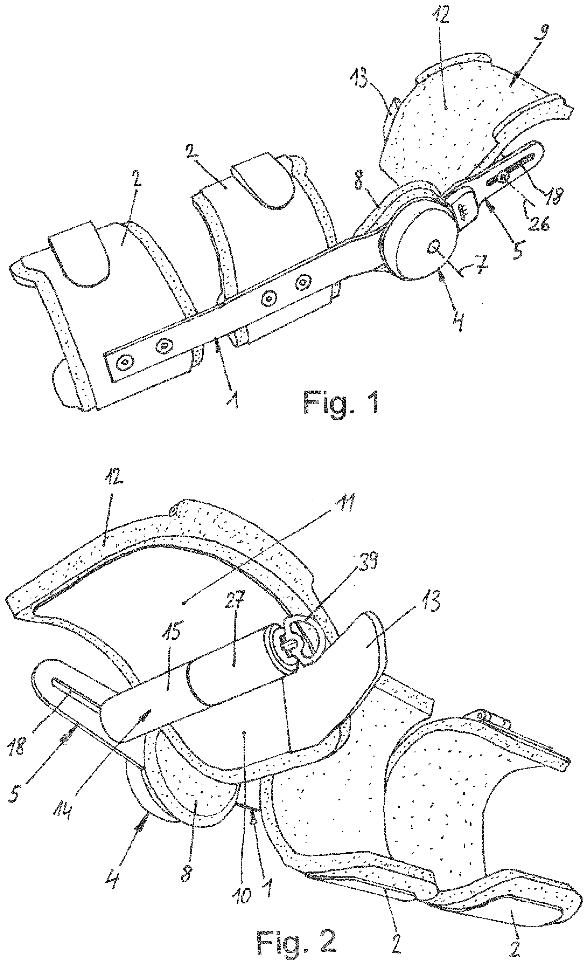

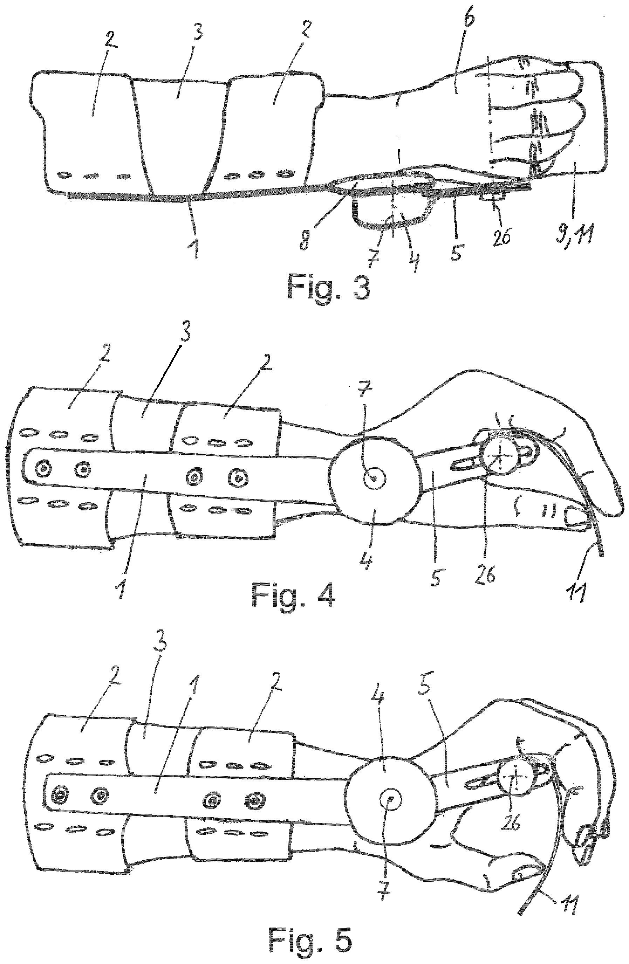

Wie aus den Fig. 1 bis 5 ersichtlich, weist die Handgelenksorthese einen proximalen Schienenarm 1 auf, der mittels gepolsterter Halbschalen 2 und nicht dargestellter, an den Halbschalen 2 befestigter Haltegurte seitlich an einem Unterarm 3 befestigt werden kann.

Das distale Ende des proximalen Schienenarms 1 ist mittels eines Drehgelenks 4 gelenkig mit einem distalen Schienenarm 5 verbunden, der sich seitlich neben der Hand 6 erstreckt.

Mittels des Drehgelenks 4 ist der distale Schienenarm 5 um eine Schwenkachse 7 schwenkbar, die im Bereich des Handgelenks liegt. Das Drehgelenk 4 weist weiterhin üblicherweise Mittel auf, mit denen der mögliche Schwenkbereich des distalen Schienenarms 5 relativ zum proximalen Schienenarm 1 begrenzt und verändert werden kann. Weiterhin weist im Fall einer dynamischen Redressionsorthese das Drehgelenk 4 auch eine Krafterzeugungseinrichtung, insbesondere in der Form einer Spiralfeder, auf, um auf den distalen Schienenarm 5 eine Vorspannkraft aufzubringen, welche den distalen Schienenarm 5 in eine Drehrichtung um die Schwenkachse 7 herum drängt. Ein derartiges Drehgelenk ist beispielsweise in der EP 0 841 044 A1 näher beschrieben. Im Rahmen der vorliegenden Erfindung ist eine derartige Krafterzeugungseinrichtung jedoch nicht unbedingt erforderlich.

Aus den Fig. 1 bis 3 ist ersichtlich, dass die Schienenarme 1, 5 und das Drehgelenk 4 auf einer Seite des Unterarms bzw. der Hand angeordnet sind. Im Rahmen der Erfindung ist es jedoch auch möglich, auf beiden Seiten des Unterarms bzw. der Hand Schienenarme 1, 5 und Drehgelenke 4 vorzusehen.

Die Schienenarme 1, 5 sind im Bereich des Drehgelenks 4 mittels eines Polsterkissens 8 zum Handgelenk hin gepolstert.

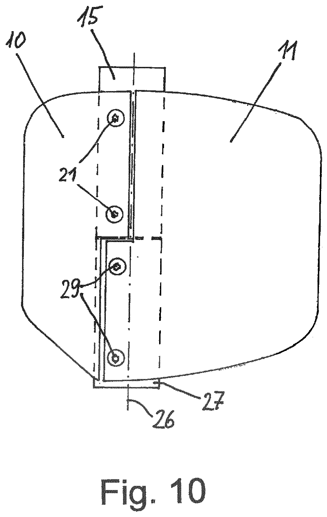

Am distalen Schienenarm 5 ist eine Handauflageeinrichtung 9 befestigt, die zum Auflegen der Hand, d.h. des Handballens und der Finger, dient. Die Handauflageeinrichtung 9 umfasst, wie aus den Fig. 2 und 10 ersichtlich, zwei voneinander getrennte, formstabile Handstützelemente, wobei ein Handstützelement als Handballenstützelement 10 zum Stützen des Handballens und das andere Handstützelement als Fingerstützelement 11 zum Stützen des Zeige-, Mittel-, Ring- und kleinen Fingers ausgebildet ist. Zweckmäßigerweise besteht das Fingerstützelement 11 aus einem gebogenen Plattenelement, während es sich beim Handballenstützelement 10 auch um ein zumindest überwiegend ebenes Plattenelement handeln kann. Zweckmäßigerweise bestehen diese Handstützelemente aus stabilen Metallblechen. Das Handballenstützelement 10 bildet zusammen mit dem Fingerstützelement 11 eine weitgehend zusammenhängende Fläche, welche mindestens die Größe der Handfläche hat.

Aus den Fig. 1 und 2 ist ersichtlich, dass das Handballenstützelement 10 und Fingerstützelement 11 von einer zusammenhängenden Polsterauflage 12 überdeckt sind, die beispielsweise aus Schaumstoff bestehen kann.

Wie aus Fig. 2 ersichtlich, kann am Handballenstützelement 10 weiterhin ein formstabiles Daumenstützelement 13 zum Aufliegen des Daumens befestigt sind. Ein derartiges Daumenstützelement 13 ist jedoch nicht unbedingt erforderlich.

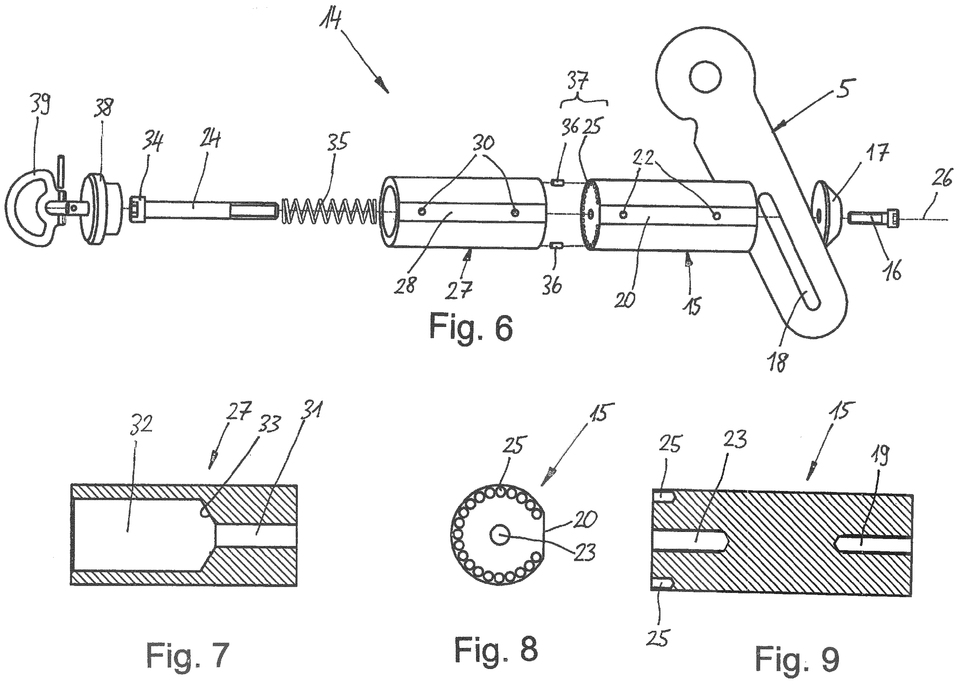

Die Handauflageeinrichtung 9 ist mittels einer Drehlagereinrichtung 14 verstellbar am distalen Schienenarm 5 befestigt. Die Drehlagereinrichtung 14 ist in Fig. 6 in Explosionsdarstellung dargestellt und umfasst ein erstes Lagerteil 15, das mittels einer Schraube 16 und einer Unterlegscheibe 17 an der distalen Schiene 5 befestigt werden kann.

Um den Abstand zwischen dem ersten Lagerteil 15 und der Schwenkachse 7 des Drehgelenks 4 variieren zu können, weist der distale Schienenarm 5 ein in seiner Längsrichtung verlaufendes Langloch 18 auf, durch das die Schraube 16 hindurchgeführt wird. Die Schraube 16 ist in eine axiale, zentrale Gewindebohrung 19 (Fig. 9) einschraubbar, die in einem Endbereich des ersten Lagerteils 15 vorgesehen ist. Die distale Schiene 5 erstreckt sich dabei zwischen einer Stirnseite des ersten Lagerteils 15 und der Unterlegscheibe 17 hindurch, so dass nach dem Festziehen der Schraube 16 das erste Lagerteil 15 drehfest und unverschiebbar an der distalen Schiene 5 festgelegt ist.

Das erste Lagerteil 15 weist weiterhin auf einer Seite eine über seine gesamte Länge verlaufende Abflachung 20 auf, die zum Aufsetzen eines Endbereichs des Handballenstützelements 10 dient. Dieser Endbereich des Handballenstützelements 10 wird, wie aus Fig. 10 ersichtlich, am ersten Lagerteil 15 mittels Schrauben 21 festgeschraubt, die in Schraubenlöcher 22 (Fig. 6) des ersten Lagerteils 15 eingeschraubt werden.

In dem zum distalen Schienenarm 5 entfernten Endbereich weist das erste Lagerteil 15 eine zentrale, axiale Gewindebohrung 23 auf. Diese Gewindebohrung 23 dient zum Einschrauben und Verankern einer zentralen Schraube 24, die in axialer Richtung über das erste Lagerteil 15 vorsteht. Weiterhin weist das erste Lagerteil 15 zu seiner Stirnseite hin offene Rastvertiefungen 25 in Form von Löchern auf, die in einem Lochkreis (Fig. 8) eng nebeneinanderliegend und in einem regelmäßigen Winkelabstand um die Mittelachse 26 der Drehlagereinrichtung 14 herum angeordnet sind. Die Rastvertiefungen 25 erstrecken sich von der Stirnseite des ersten Lagerteils 15 in Axialrichtung, d.h. parallel zur Mittelachse 26, ein kleines Stück in das Innere des ersten Lagerteils 15 hinein.

Die Drehlagereinrichtung 14 umfasst ferner ein zweites Lagerteil 27, das hülsen- oder hohlzylinderförmig ausgebildet ist und eine gleiche oder ganz ähnliche Außenkontur wie das erste Lagerteil 15 aufweist. Das zweite Lagerteil 27 dient zum Befestigen des Fingerstützelements 11. Hierzu weist das zweite Lagerteil 27 auf einer Seite eine sich über seine gesamte Länge erstreckende Abflachung 28 auf, auf die ein Endbereich des Fingerstützelements 11 aufgesetzt wird. Dieser Endbereich des Fingerstützelements 11 wird am zweiten Lagerteil 27 mittels Schrauben 29 (Fig. 10) festgeschraubt, die in Schraubenlöcher 30 (Fig. 6) des zweiten Lagerteils 27 eingeschraubt werden.

Wie aus Fig. 7 ersichtlich, weist das zweite Lagerteil 27 eine axial durchgehende, im Durchmesser gestufte Axialbohrung auf. Ein im Durchmesser verringerter Bohrungsabschnitt 31 weist einen Durchmesser auf, der nur geringfügig größer ist als der Durchmesser der Schraube 24, so dass der Schaft der Schraube 24 mit geringem Spiel hindurchgeführt werden kann. Der Bohrungsabschnitt 31 dient als Führungs- oder Lagerabschnitt zur längsverschiebbaren und drehbaren Lagerung des zweiten Lagerteils 27 längs bzw. an der Schraube 24.

An den Bohrungsabschnitt 31 schließt über eine Durchmesserstufe 33 ein im Durchmesser vergrößerter Innenraum 32 an. Im montierten Zustand der Drehlagereinrichtung 14, d.h. wenn das zweite Lagerteil 27 fluchtend zum ersten Lagerteil 15 angeordnet und die zentrale Schraube 24 über den Innenraum 32 und den Bohrungsabschnitt 31 des zweiten Lagerteils 27 hindurch in die Gewindebohrung 23 des ersten Lagerteils 15 eingeschraubt ist, erstreckt sich die zentrale Schraube 24 mit ihrem Schraubenkopf 34 über den Bohrungsabschnitt 31 hinaus ein Stück weit in den Innenraum 32 hinein. Die zentrale Schraube 24 ist hierbei durch eine Feder 35 hindurchgeführt, die im vorliegenden Beispiel als Druckfeder ausgebildet ist und sich mit einem Ende am Schraubenkopf 34 und mit ihrem gegenüberliegenden Ende an der Durchmesserstufe 33 des zweiten Lagerteils 27 abstützt. Die Feder 35 übt auf das zweite Lagerteil 27 eine axial gerichtete Vorspannkraft aus, welche das zweite Lagerteil 27 stirnseitig gegen das erste Lagerteil 15 drückt.

Wie weiterhin aus Fig. 6 ersichtlich, sind am zweiten Lagerteil 27 weiterhin zwei Stifte 36 befestigt, die stirnseitig über das zweite Lagerteil 27 vorstehen. Die Stifte 36 weisen zur Mittelachse 26 den gleichen Abstand wie die Rastvertiefungen 25 auf und sind derart ausgebildet, dass sie sich in hierzu fluchtende Rastvertiefungen 25 des ersten Lagerteils 15 hinein erstrecken, wenn die beiden Lagerteile 15, 27 stirnseitig dicht aneinander liegen. Soll das zweite Lagerteil 27 und damit das daran befestigte Fingerstützelement 11 relativ zum ersten Lagerelement 15 und damit zum Handballenstützelement 10 verdreht werden, kann das zweite Lagerteil 27 entgegen der Vorspannkraft der Feder 35 in Axialrichtung vom ersten Lagerteil 15 entfernt und um die zentrale Schraube 24 herum, d.h. um die Mittelachse 26, in die gewünschte Drehwinkelstellung verdreht werden, bis die Stifte 36 mit anderen Rastvertiefungen 25 fluchten. Wird anschließend das zweite Lagerteil 27 losgelassen, wird das zweite Lagerteil 27 durch die Feder 35 wieder an das erste Lagerteil 15 gedrückt, worauf die Stifte 36 in die fluchtenden Rastvertiefungen 25 eingreifen. Die Stifte 36 bilden damit zusammen mit den Rastvertiefungen 25 eine Rasteinrichtung 37, mit welcher das zweite Lagerteil 27 in unterschiedlichen Drehwinkelstellungen am ersten Lagerteil 15 arretiert werden kann.

Auf das zweite Lagerteil 27 ist stirnseitig eine Verschlusskappe 38 aufgesetzt, die fest mit dem zweiten Lagerteil 27 verbunden ist. An der Verschlusskappe 38 ist ein ringförmiges Handhabungsteil 39 befestigt, das mit den Fingern ergriffen werden kann und zum Ziehen des zweiten Lagerteils 27 entgegen der Vorspannkraftfeder 35 dient.

Es ist ersichtlich, dass durch einfaches Ziehen am Handhabungsteil 39 das zweite Lagerteil 27 zusammen mit dem Fingerstützelement 11 in axialer Richtung vom zweiten Lagerteil 15 entfernt, in gewünschter Weise gedreht und in unterschiedliche Drehwinkelstellungen gebracht werden kann. Wird keine Zugkraft mehr aufgebracht, erfolgt wieder eine automatische Verriegelung des Lagerteils 27 und damit des Fingerstützelements 11 am ersten Lagerteil 15.

Im Rahmen der Erfindung ist eine Vielzahl von Variationen möglich. Beispielsweise ist es möglich, anstelle der beschriebenen Feder 35 eine andere Krafterzeugungseinrichtung zur Erzeugung einer axialen Vorspannkraft für das zweite Lagerteil 27 vorzusehen, beispielsweise ein elastisches Zugband, das einerseits am zweiten Lagerteil 27 und andererseits am ersten Lagerteil 15 befestigt ist. Die Rasteinrichtung 37 kann ohne weiteres derart modifiziert werden, dass die Stifte 36 nicht am zweiten Lagerteil 27, sondern am ersten Lagerteil 15 befestigt sind, während die Rastvertiefungen 25 am zweiten Lagerteil 27 angeordnet sind. Anstelle von zwei Stiften 36 kann auch eine andere Anzahl von Stiften, beispielsweise lediglich ein Stift, vorgesehen sein. Weiterhin sind anstelle von Stiften 36 auch Zähne denkbar, die in dazu passende Kerben oder Ausnehmungen im gegenüberliegenden Lagerteil eingreifen.

Obwohl die werkzeuglose Verriegelung des zweiten Lagerteils 27 am ersten Lagerteil 15 mittels der Feder 35 und der Rasteinrichtung 37 besonders vorteilhaft ist, ist es im Rahmen der Erfindung auch möglich, das zweite Lagerteil 27 allein mittels Befestigungsschrauben am ersten Lagerteil 15 zu befestigen, die in einen Lochkreis bildende, unterschiedlich positionierte Schraubenlöcher des ersten Lagerteils 15 einschraubbar sind.

Das Fingerstützelement 11 kann insbesondere dann, wenn nur ein bis drei Finger gestützt werden müssen, zusammen mit dem Handballenstützelement 10 auch kleiner als die Handfläche ausgebildet sein.

## Patentansprüche

1. Handgelenksorthese mit
- einem am Unterarm (3) befestigbaren proximalen Schienenarm (1),
- einem distalen Schienenarm (5),
- einem Drehgelenk (4) zur gelenkigen Verbindung des distalen Schienenarms (5) mit dem proximalen Schienenarm (1),
- einer am distalen Schienenarm (5) befestigten Handauflageeinrichtung (9), die zwei voneinander getrennte, formstabile Handstützelemente aufweist, wobei ein Handstützelement als Handballenstützelement (10) zum Stützen des Handballens und das andere Handstützelement als Fingerstützelement (11) zum Stützen mindestens eines Fingers ausgebildet ist,
- wobei die Handauflageeinrichtung (9) eine Drehlagereinrichtung (14) aufweist, mit der die beiden Handstützelemente gelenkig miteinander verbunden sind **dadurch gekennzeichnet, dass** die Drehlagereinrichtung (14) ein erstes Lagerteil (15), das am distalen Schienenarm (5) befestigt ist und das Handballenstützelement (10) trägt, und ein zweites Lagerteil (27) umfasst, das drehbar am ersten Lagerteil (15) gehaltert ist und das Fingerstützelement (11) trägt,
- und wobei die Handauflageeinrichtung (9) eine Arretiereinrichtung zum Arretieren des Fingerstützelementes (11) relativ zum Handballenstützelement (10) in einer bestimmten Drehwinkelstellung aufweist, wobei das zweite Lagerteil (27) der Drehlagereinrichtung (14) mittels der Kraft einer Krafterzeugungseinrichtung in eine Verriegelungsstellung vorgespannt ist, in der das zweite Lagerteil (27) in Druckkontakt mit dem ersten Lagerteil (15) und relativ zum ersten Lagerteil (15) drehverriegelt ist, und dass das zweite Lagerteil (27) entgegen der Vorspannkraft der Krafterzeugungseinrichtung in eine Freigabestellung bewegbar ist, in der das zweite Lagerteil (27) relativ zum ersten Lagerteil (15) drehbar ist.

2. Handgelenksorthese nach Anspruch 1, **dadurch gekennzeichnet, dass** das am distalen Schienenarm (5) befestigte erste Lagerteil (15) das Handballenstützelement (10) trägt.

3. Handgelenksorthese nach Anspruch 2, **dadurch gekennzeichnet, dass** das zweite Lagerteil (27) fluchtend zum ersten Lagerteil (15) angeordnet ist.

4. Handgelenksorthese nach Anspruch 2 oder 3, **dadurch gekennzeichnet, dass** der distale Schienenarm (5) ein sich in seiner Längsrichtung erstreckendes Langloch (18) oder eine Mehrzahl von Bohrungen zur Befestigung des ersten Lagerteils (15) in unterschiedlichen Abständen zum Drehgelenk (4) aufweist.

5. Handgelenksorthese nach einem der Ansprüche 2 bis 4, **dadurch gekennzeichnet, dass** das erste und das zweite Lagerteil (15, 27) zumindest überwiegend zylinderförmig ausgebildet sind.

6. Handgelenksorthese nach einem der Ansprüche 2 bis 5, **dadurch gekennzeichnet, dass** das erste und zweite Lagerteil (15, 27) den gleichen Außendurchmesser aufweisen.

7. Handgelenksorthese nach einem der Ansprüche 2 bis 6, **dadurch gekennzeichnet, dass** die Krafterzeugungseinrichtung aus einer Feder besteht.

8. Handgelenksorthese nach einem der Ansprüche 2 bis 7, **dadurch gekennzeichnet, dass** das zweite Lagerteil (27) längsverschiebbar an einem Führungsstift geführt ist, der am ersten Lagerteil (15) befestigt ist und sich in das zweite Lagerteil (27) hinein erstreckt.

9. Handgelenksorthese nach Anspruch 8, **dadurch gekennzeichnet, dass** der Führungsstift aus einer zentralen Schraube (24) besteht, die in das erste Lagerteil (15) eingeschraubt ist und einen Schraubenkopf (34) aufweist, der einen Anschlag für ein erstes Ende der Feder (35) bildet, während ein zweites Ende der Feder (35) im oder am zweiten Lagerteil (27) abgestützt ist.

10. Handgelenksorthese nach Anspruch 9, **dadurch gekennzeichnet, dass** das zweite Lagerteil (27) hülsenförmig ausgebildet ist und der Schraubenkopf (34) und die Feder (35) im Inneren des zweiten Lagerteils (27) angeordnet sind.

11. Handgelenksorthese nach einem der Ansprüche 2 bis 10, **dadurch gekennzeichnet, dass** die Arretiereinrichtung eine Rasteinrichtung (37) umfasst, die mindestens einen Stift (36) oder Zahn aufweist, der am ersten oder zweiten Lagerteil (15, 27) angeordnet ist und in unterschiedlich positionierte Rastvertiefungen (25) eingeführt werden kann, die am gegenüberliegenden Lagerteil (15, 27) angeordnet sind.

12. Handgelenksorthese nach Anspruch11, **dadurch gekennzeichnet, dass** die Rastvertiefungen (25) in Umfangsrichtung des ersten oder zweiten Lagerteils (15, 27) in einem regelmäßigen Winkelabstand angeordnet sind.

## Claims

1. Method for producing a laminate for protective clothing for protection against heat and flames, wherein the laminate comprises at least one textile layer that consists of flame-resistant fibres made of synthetic polymers and cellulose fibres, wherein the synthetic polymers are selected from the group consisting of para-aramid, meta-aramid, aromatic PES, PBI, melamine rein, novoloid, modacrylic and FR polyamide, either in pure form or in a mixture thereof, and wherein the textile layer is subjected to a treatment step in which at least one fibre component is extracted from the textile layer at least in part.

2. Method according to claim 1, **characterised in that** at least one fibre component is extracted from the flame-resistant textile layer by means of discharge printing.

3. Method according to claim 2, **characterised in that** a discharge paste is applied to the flame-resistant textile layer, which discharges the cellulose fibres when the discharge paste is activated at temperatures above 150 °C.

4. Method according to any of claims 1 to 3, **characterised in that** the flame-resistant textile layer comprises a water-soluble fibre component, and **in that** the flame-resistant textile layer is subjected to a treatment step in which the water-soluble fibre component is washed out of the flame-resistant textile layer at least in part.

5. Method according to claim 4, **characterised in that** the water-soluble fibre component consists of polyvinyl alcohol fibres, and **in that** the polyvinyl alcohol fibres are washed out by means of a slightly acidic aqueous solution having a pH of between 4 and 5.

6. Method according to claim 1, **characterised in that** the flame-resistant textile layer comprises both a first fibre component, which can be extracted from the flame-resistant textile layer by means of a discharge paste, and a second water-soluble water component, which can be washed out of the flame-resistant textile layer by means of an aqueous solution, the first fibre component being extracted from the textile layer by means of a discharge printing treatment and the second water-soluble fibre component being extracted from said textile layer by means of a washing-out treatment.

7. Method according to claim 6, **characterised in that** the first fibre component treated with the discharge paste and the second water-soluble fibre component are extracted at the same time in a washing-out step.

8. Method according to claim 6, **characterised in that** the first fibre component treated with the discharge paste and the second water-soluble fibre component are extracted at the same time during a dyeing process.

9. Flame-resistant textile material for protective clothing for protection against heat and flames, which is produced in accordance with a method according to any of claims 1 to 8.

10. Flame-resistant textile material according to claim 9, **characterised in that** at least 10-60 % of the textile layer consists of cellulose fibres, 10-60 % consists of water-soluble polyvinyl alcohol fibres and 10-60 % consists of fibres from the group consisting of flame-resistant fibres made of synthetic polymers.

11. Flame-resistant textile material according to either claim 9 or claim 10, **characterised in that** the textile material comprises a plurality of textile layers, a fibre component being extracted from at least one textile layer by means of discharge printing, and a water-soluble fibre component being washed out of at least one other textile layer.

12. Flame-resistant protective clothing for protection against heat and flames, at least predominantly made of a flame-resistant textile material according to any of claims 9 to 11.

## Revendications

1. Procédé pour réaliser un stratifié pour vêtements de protection destinés à la protection contre la chaleur et les flammes,
le stratifié comprenant au moins une couche textile qui est constituée de fibres difficilement inflammables en polymères synthétiques et en fibres de cellulose, les polymères synthétiques étant choisis parmi le groupe comprenant para-aramide, méta-aramide, PES aromatiques, PBI, résine de mélamine, novoloïde, modacryle, polyamide FR, en forme pure ou un mélange de ceux-ci, et la couche textile étant soumise à une étape de traitement dans laquelle au moins une composante fibreuse est extraite au moins partiellement hors de la couche textile.

2. Procédé selon la revendication 1,
**caractérisé en ce que**
au moins une composante fibreuse est extraite hors de la couche textile difficilement inflammable par attaque chimique sous pression.

3. Procédé selon la revendication 2,
**caractérisé en ce que**
une pâte d'attaque chimique est appliquée sur la couche textile difficilement inflammable, pâte qui attaque les fibres de cellulose lorsque la pâte d'attaque chimique est activée à des températures supérieures à 150 °C.

4. Procédé selon l'une des revendications 1 à 3,
**caractérisé en ce que**
la couche textile difficilement inflammable inclut une composante fibreuse soluble à l'eau, et **en ce que**
la couche textile difficilement inflammable est soumise à une étape de traitement dans laquelle la composante fibreuse soluble à l'eau est extraite par lavage au moins partiellement hors de la couche textile difficilement inflammable.

5. Procédé selon la revendication 4,
**caractérisé en ce que**
la composante fibreuse soluble à l'eau est constituée de fibres d'alcool polyvinylique, et **en ce que**
l'extraction par lavage des fibres d'alcool polyvinylique est effectuée au moyen d'une solution aqueuse légèrement acide qui présente un pH entre 4 et 5.

6. Procédé selon la revendication 1,
**caractérisé en ce que**
la couche textile difficilement inflammable inclut aussi bien une première composante fibreuse qui peut être extraite hors de la couche textile difficilement inflammable au moyen d'une pâte d'attaque chimique, qu'une seconde composante aqueuse soluble à l'eau qui peut être extraite par lavage hors de la couche textile difficilement inflammable au moyen d'une solution aqueuse, et aussi bien la première composante fibreuse est extraite hors de la couche textile par traitement par d'attaque chimique sous pression que la seconde composante fibreuse soluble à l'eau est extraite hors de ladite couche textile par le traitement par lavage.

7. Procédé selon la revendication 6,
**caractérisé en ce que**
l'extraction de la première composante fibreuse traitée à la pâte d'attaque chimique et de la seconde composante fibreuse aqueuse s'effectue simultanément dans une étape d'extraction par lavage.

8. Procédé selon la revendication 6,
**caractérisé en ce que**
l'extraction de la première composante fibreuse traitée à la pâte d'attaque chimique et de la seconde composante fibreuse aqueuse s'effectue simultanément pendant un processus de coloration.

9. Matériau textile difficilement inflammable pour vêtements de protection destinés à la protection contre la chaleur et les flammes, réalisé par un procédé selon l'une des revendications 1 à 8.

10. Matériau textile difficilement inflammable selon la revendication 9,
**caractérisé en ce que**
la couche textile est constituée d'au moins 10 à 60 % de fibres de cellulose, de 10 à 60 % de fibres d'alcool polyvinylique solubles à l'eau et de 10 à 60 % de fibres du groupe des fibres difficilement inflammables en polymères synthétiques.

11. Matériau textile difficilement inflammable selon la revendication 9 ou 10,
**caractérisé en ce que**
le matériau textile comprend plusieurs couches textiles, et
dans au moins une couche textile, une composante fibreuse est extraite par attaque chimique sous pression, et
dans au moins une autre couche textile, une composante fibreuse soluble à l'eau est extraite par lavage.

12. Vêtement de protection difficilement inflammable destiné à la protection contre la chaleur et les flammes, constitué au moins majoritairement en un matériau textile difficilement inflammable selon l'une des revendications 9 à 11.
